# EUROPEAN PATENT SPECIFICATION

(11) **EP 3 505 049 B1**
(45) Date of publication and mention of the grant of the patent: **24.09.2025**
(21) Application number: 17211116.3
(22) Date of filing: 29.12.2017
(51) Int. Cl.: A61B 5/00, H04B 13/00

(54) **INTRA-BODY COMMUNICATION APPARATUS AND METHOD**
INTRAKORPORALE KOMMUNIKATIONSVORRICHTUNG UND VERFAHREN
PROCÉDÉ ET APPAREIL DE COMMUNICATION INTRA-CORPORELLE

(43) Date of publication of application: 03.07.2019
(73) Proprietor: Nokia Technologies Oy, 02610 Espoo (FI)
(72) Inventor: SEPPONEN, Raimo, 00530 Helsinki (FI); LAAKSO, Ilkka, 00500 Helsinki (FI); PALVA, Lauri, 01300 Vantaa (FI); SAASTAMOINEN, Joel, 02250 Espoo (FI); LINNAVUO, Matti, 02210 Espoo (FI)
(74) Representative: Whiting, Gary

(56) References cited:
- WO-A1-2017/035143
- US-A1- 2008 306 359
- US-B2- 7 480 492
- TANHA MILAD: "Eddy Current Sensor for Tissue Conductivity measurement", 1 February 2013 (2013-02-01), XP055462449, Retrieved from the Internet <URL:http://www.diva-portal.org/smash/get/diva2:607825/FULLTEXT02.pdf> [retrieved on 20180323]
- G.A MOUCHAWAR ET AL: "Magnetic stimulation of excitable tissue: calculation of induced eddy-currents with a three-dimensional finite-element model", IEEE TRANSACTIONS ON MAGNETICS, 1 January 1993 (1993-01-01), pages 3355 - 3357, XP055462187, Retrieved from the Internet <URL:http://ieeexplore.ieee.org/ielx3/5091/13885/00642593.pdf?tp=&arnumber=642593&isnumber=13885> [retrieved on 20180323], DOI: 10.1109/20.281174
- YI LV ET AL: "Comparison of Measurement Method for Eddy Current Signal of Biological Tissue in Magnetic Induction Tomography", BIOINFORMATICS AND BIOMEDICAL ENGINEERING, (ICBBE) 2011 5TH INTERNATIONAL CONFERENCE ON, 1 January 2011 (2011-01-01), pages 1 - 4, XP055462394, ISBN: 978-1-4244-5088-6, DOI: 10.1109/icbbe.2011.5780339
- J. HELLER ET AL: "Auto-Tuned Induction Coil Conductivity Sensor for In-Vivo Human Tissue Measurements", MEASUREMENT SCIENCE REVIEW, vol. 9, no. 6, 1 January 2009 (2009-01-01), XP055462671, DOI: 10.2478/v10048-009-0027-1

## Description

### Field

Example embodiments disclosed herein relate to an intra-body communication apparatus and method in which signals are transmitted at least partially using a body as a transmission medium.

### Background

Many sensors are available that measure human or animal characteristics, such as heart rate, blood pressure, oxygen saturation, oxygen levels etc. Wireless solutions have been developed to connect such sensors to, for example, a controller unit for collating data. A number of problems exist with known wireless solutions.

Measurement sensors may be separate devices connected to the body. The body may be the common factor for all sensors and that body may be used for transmitting information, such as sensor data. In the context of healthcare, the use of multiple sensors for patient monitoring in which the human or animal body is used as a data transmission path can reduce the time spent in hospitals and provide for recovery without the risk of hospital infections. Similarly, older or infirm patients can continue to live in their own homes more safely.

There remains a need for alternative and improved solutions in this field.

The document "Eddy Current Sensor for Tissue Conductivity Measurement" by Tanha Milad, 1 February 2013 (2013-02-01), XP055462449, [retrieved on 2018-03-23], describes a new application of eddy current sensor, one of basics of non-destructive testing, NDT, in biomedical engineering. For medical purposes, conductivity of tissue or tissue equivalent samples is evaluated and compared with other specimens via sensors based on eddy current techniques. To reach the aim, the electronic setups are evaluated via measurements on targets with predetermined conductivities. Eddy current was induced in targets by magnetic field of the coil and that eddy current generated a secondary magnetic field which opposed the primary magnetic field and affect the inductance and consequently the impedance of the coil. The measured output (RMS-value or its amplitude) from bridge circuit is correlated to the change in coil's inductance and impedance and those are proportional to change in conductivity of the targets. Prominent sensor characteristics such as quality factor, inductance and operating frequency considered to be high enough and desired sensor dimensions are assessed.

International Patent Publication WO2017035143A1 provides a body area network that uses a near magnetic field for communications. A first coil is configured to be worn on a body portion of a human. A transmitter drives the first coil to generate a magnetic body field through the first magnetic coil. A second coil couples to the signal transmitted via the first coil matched to the magnetic field and first coil. A receiver for receiving the signal from the second coil. A method for establishing network communications using the human body as a magnetic field includes associating a transmitter coil with a portion of a human body, the transmitter coil being configured to couple to a receiver coil in a near field of the human body; driving the transmitter coil to generate a magnetic near field around the human body; and coupling to the magnetic near field with a receiver coil.

US Patent Application US2008/0306359 describes a method for exchanging information with implantable medical devices, where two implantable devices communicate with each other using quasi-electrostatic signal transmission in a long wavelength/low frequency electromagnetic band, with the patient's body acting as a conductive medium.

### Summary

The object of the present invention is achieved with a method or apparatus according to the independent claims.

In a first aspect, this specification describes a method comprising: receiving or generating a signal for transmission, wherein the signal for transmission is based on an input signal; and converting the signal for transmission into a variable magnetic field directed towards a human or animal body, wherein the variable magnetic field induces eddy currents in order to generate an electrical current signal in the human or animal body. The variable magnetic field may be generated using a magnetic coil. The variable magnetic field may be an oscillating magnetic field.

The invention comprises generating the signal for transmission by encoding the input signal.

The first aspect may further comprise receiving the input signal from one or more sensors.

The first aspect may further comprise using receiver electrodes worn on the human or animal body to measure an electrical potential difference in the human or animal body, the electrical potential difference being indicative of the electrical current signal. The first aspect may further comprise amplifying the measured electrical potential difference.

In a second aspect, this specification describes an apparatus configured to perform any method as described with reference to the first aspect.

In a third aspect, this specification describes computer-readable instructions which, when executed by computing apparatus, cause the computing apparatus to perform any method as described with reference to the first aspect.

In a fourth aspect, this specification describes an apparatus comprising: an input for receiving an input signal; and a transmitter for converting a signal based on the input signal into a variable magnetic field, wherein, in use, the variable magnetic field induces eddy currents in order to generate an electrical current signal in a human or animal body. The apparatus according to the invention comprises a transmitter circuitry for encoding the input signal.

The apparatus may further comprise one or more sensors for providing the input signal. By way of example, the one or more sensors may comprise one or more of a heart rate sensor, a blood pressure sensor and an oxygen level sensor.

The apparatus may be configured to be worn on the human or animal body.

Alternatively, or in addition, the apparatus may be configured to be either totally or partially implanted inside the human or animal body.

The fourth aspect may further comprise a receiver comprising receiver electrodes configured to measure an electrical potential difference in the human or animal body, wherein the electrical potential difference is indicative of the electrical current signal. The receiver electrodes may, for example, be capacitive electrodes.

In a fifth aspect, this specification describes a computer-readable medium having computer-readable code stored thereon, the computer readable code, when executed by at least one processor, causing performance of: receiving or generating a signal for transmission, wherein the signal for transmission is based on an input signal; and converting the signal for transmission into a variable magnetic field directed towards a human or animal body, wherein the variable magnetic field induces eddy currents in order to generate an electrical current signal in the human or animal body.

In a sixth aspect, this specification describes an apparatus comprising: at least one processor, and at least one memory including computer program code which, when executed by the at least one processor, causes the apparatus to receive or generate a signal for transmission, wherein the signal for transmission is based on an input signal; and convert the signal for transmission into a variable magnetic field directed towards a human or animal body, wherein the variable magnetic field induces eddy currents in order to generate an electrical current signal in the human or animal body.

In a seventh aspect, this specification describes an apparatus comprising: means for receiving or generating a signal for transmission, wherein the signal for transmission is based on an input signal; and means for converting the signal for transmission into a variable magnetic field directed towards a human or animal body, wherein the variable magnetic field induces eddy currents in order to generate an electrical current signal in the human or animal body.

### Brief description of the drawings

Embodiments will now be described, by way of non-limited examples, with reference to the following schematic drawings, of which:
Figure 1 shows an apparatus in accordance with an example embodiment;
Figure 2 show an apparatus in accordance with an example embodiment;
Figure 3 show a system in accordance with an example embodiment;
Figures 4 and 5 show simulation results for example embodiments;
Figure 6 is a block diagram of a system in accordance with an example embodiment;
Figure 7 is a flow chart showing an algorithm in accordance with an example embodiment.
Figure 8 shows a data signal used in an example embodiment;
Figure 9 is a diagram showing an example embodiment;
Figure 10 is a diagram demonstrating example applications of the principles described herein;
Figure 11 is a block diagram of components of a processing system in accordance with an example embodiment; and
Figures 12a and 12b show tangible media, respectively a removable memory unit and a compact disc (CD) storing computer-readable code which when run by a computer perform operations according to example embodiments.

### Detailed description

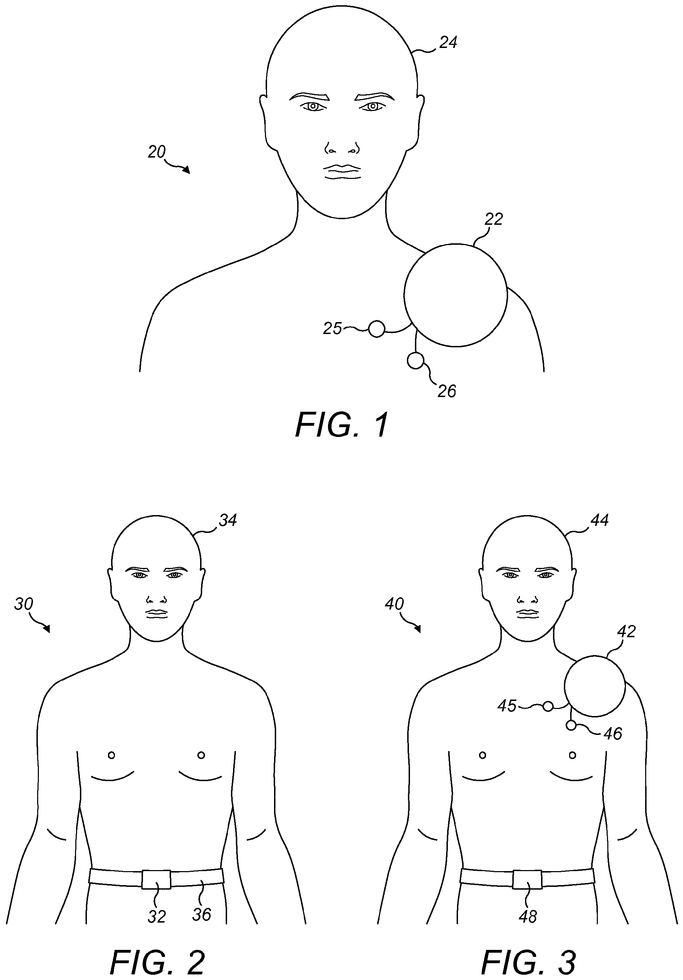

Figure 1 shows an apparatus, indicated generally by the reference numeral 20, in accordance with an example embodiment. The apparatus 20 includes a coil 22 that is used to direct a magnetic field towards a human body 24. The coil 22 includes sensor inputs 25 and 26 for receiving an input signal, for example from sensors 25 and 26 shown in Figure 1. The sensor inputs 25 and 26 can be used to provide data for transmission by the apparatus 20.

Figure 2 shows an apparatus, indicated generally by the reference numeral 30, in accordance with an example embodiment. The apparatus 30 includes a transmitter 32 that is used to direct a magnetic field towards a human body 34. The transmitter is in the form of a coil wrapped around the human body 34. The coil may be attached using a strap 36.

Figure 1 and 2 are two of many examples of ways in which a magnetic field can be generated, for example using a magnetic coil, and directed towards a human body. The magnetic field generated by the coils 22 and 32 is used to induce eddy currents in the human body 24, 34. Eddy currents are loops of electrical current induced within conductors by a changing magnetic field in the conductor. The induced eddy currents generate electric fields which may be detected using a suitable receiver circuit. In this way, signals can be transmitted using the human body 24, 34 as a volume conductor. The transmitted signals can be based on an input signal, for example from the sensor inputs 25 and 26.

A technical effect of some implementations of the apparatuses 20 and 30 is that a ground plane can, in some circumstances, be at least partially eliminated. This may make the implementation of the apparatuses 20 and 30 highly practical and may contribute towards a reduction in the risk of eavesdropping, since electrical signals can sometimes be detected in ground planes or by using electric field detecting devices through stray electric fields outside the body.

Figure 3 shows a system, indicated generally by the reference numeral 40, in accordance with an example embodiment. The system 40 includes a coil 42 that is used to direct a magnetic field towards a human body 44. The coil 42 includes sensor inputs, for example from sensors 45 and 46 shown in Figure 3. The system 40 also includes a receiver 48. As described above, the magnetic field generated by the coil 42 induces eddy currents that generate electric fields within the human body 44 that can be detected by the receiver 48. The system 40 is an example of one of many possible transmitter and receiver arrangements in accordance with the principles described herein.

In the system 40, the isolation of sensors (e.g. where sensors are on one side of a human or animal body and a controller unit is on the other side of the body) may be at least partially avoided by making use of the human body 44 (or an animal body) as a transmission channel. Furthermore, connection problems caused by walking, sleeping or other human or animal movement or activities may be at least partially prevented.

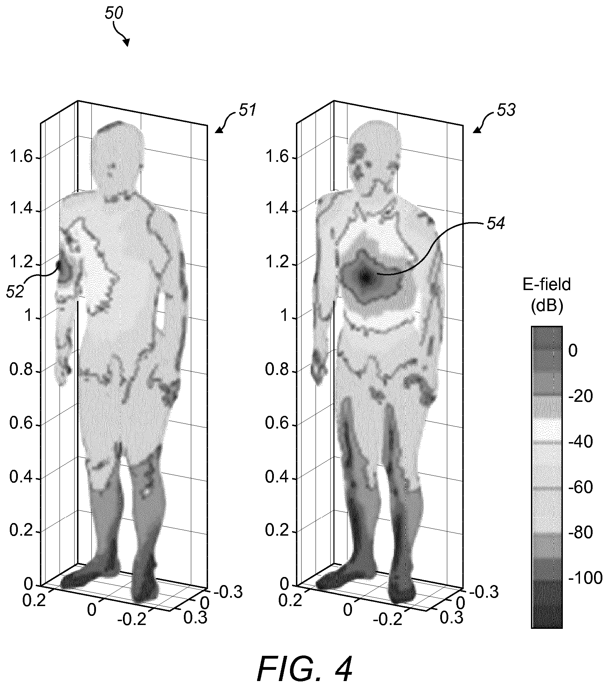

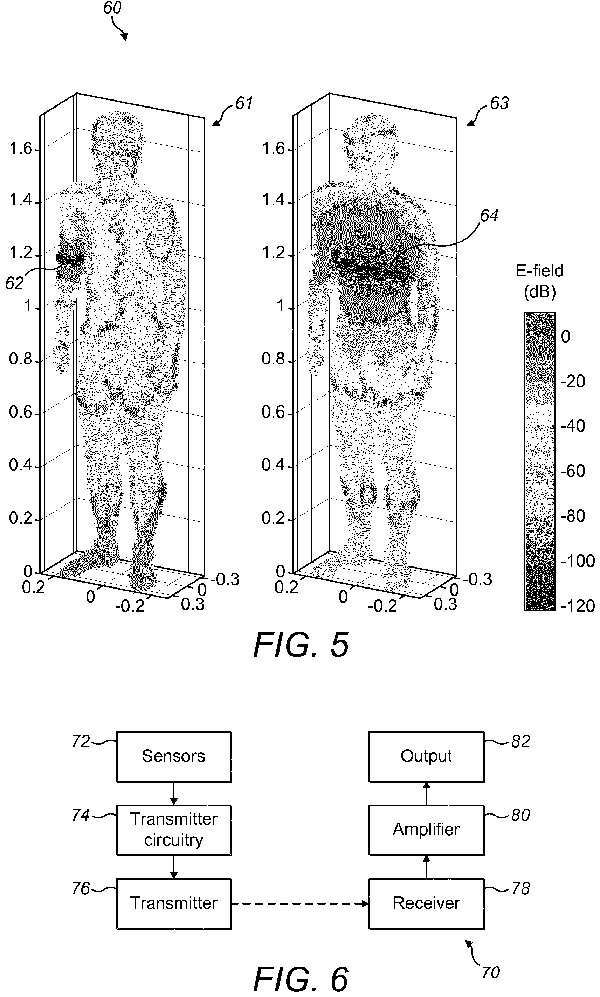

Figure 4 shows example simulation results, indicated generally by the reference numeral 50, simulating a use of the apparatus 20 described above. Similarly, Figure 5 shows example simulation results, indicated generally by the reference numeral 60, simulating a use of the apparatus 30 described above.

The simulation results 50 and 60 are simulations of induced electric fields for an adult male having a height of 173 centimetres and a weight of 53 kilograms. In each case, a magnetic transmitter (the coils 22 and 32 respectively) was simulated operating at 500 kHz.

The simulation results 50 show results of simulations in which a magnetic transmitter 22 is a round coil with a diameter of two centimetres positioned three millimetres above the skin surface of the model of the human body 24. A receiver (not shown in Figure 1) was modelled as three galvanic or capacitive electrodes in a triangle shape so that voltage in any direction can be measured. The electrodes were assumed to be connected to an amplifier that can measure a voltage of one microvolt or higher. The distance between measurement points was two centimetres.

The simulation results 50 model the attenuation of induced electric fields with the transmitter 22 placed in different positions on the body 24. A first simulation result 51 shows the transmitter placed on the upper arm of the body (as indicated by the reference numeral 52). A second simulation result 53 shows the transmitter placed in the centre of the chest of the body (as indicated by the reference numeral 54).

The simulation results 60 show results of simulations in which a magnetic transmitter 32 is a coil wrapped around the body 34, the coil being positioned three millimetres above the skin surface of the model of the human body 34. As with the simulation results 50, a receiver (not shown in Figure 2) was modelled as three galvanic or capacitive electrodes in a triangle shape so that voltage in any direction can be measured. The electrodes were assumed to be connected to an amplifier that can measure a voltage of one microvolt or higher. The distance between measurement points was two centimetres.

The simulation results 60 model the attenuation of induced electric fields with the transmitter 32 placed in different positions on the body 44. A first simulation result 61 shows the transmitter placed on the upper arm of the body (as indicated by the reference numeral 62). A second simulation result 63 shows the transmitter placed in the centre of the chest of the body (as indicated by the reference numeral 64).

The simulation results 50 and 60 map the modelled electric field around the body. The simulation results 50 suggest that with a minimum detectable electric field strength of about -80dB, the system 20 can be used to transmit data around at least the top half of the body 24 (i.e. above the waist). The simulation results 60 suggest that the system 30 can be used to transmit data further than the system 20 (for example, from the chest to the legs of the body in the second simulation result 63). In both cases, data transmission is possible.

Figure 6 is a block diagram of a system, indicated generally by the reference numeral 70, in accordance with an example embodiment. The system 70 comprises one or more sensors 72, transmitter circuitry 74, a transmitter 76, a receiver 78, an amplifier 80 and an output 82 (which may include a receiver controller of the receiver circuitry).

The one or more sensors 72 may include one or more of a heart rate sensor, a blood pressure sensor and an oxygen level sensor. Alternative or additional sensors are possible.

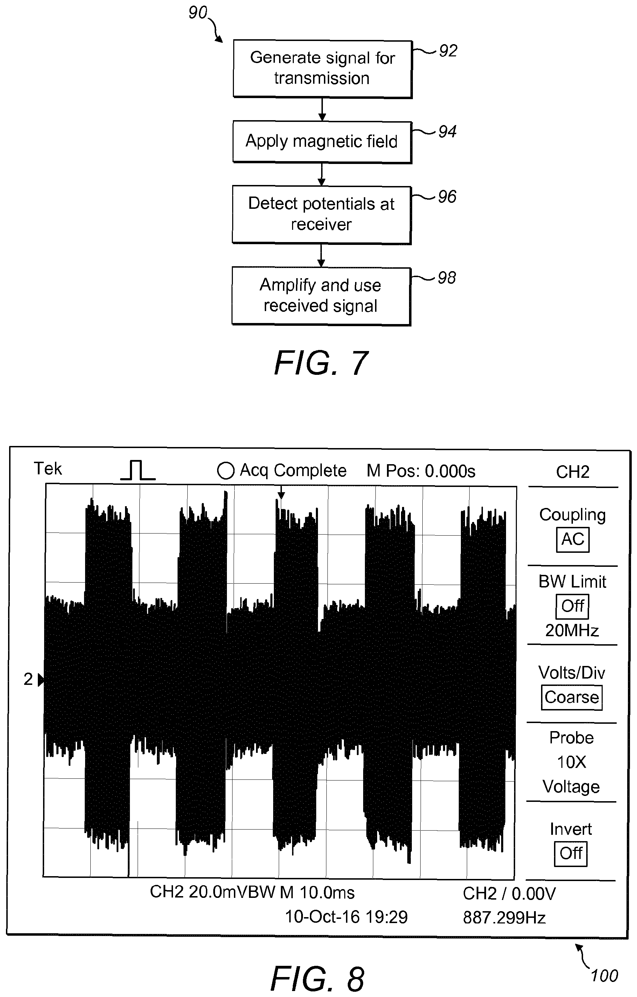

Figure 7 is a flow chart showing an algorithm, indicated generally by the reference numeral 90, in accordance with an example embodiment.

The algorithm 90 starts at operation 92 where signals for transmission are received or generated based on an input signal. The signals for transmission may be generated by the transmitter circuitry 74, for example in response to input signals received from the one or more sensors 72.

At operation 94, the transmitter 76 is used to transmit the signals received or generated in operation 92 by applying a magnetic field in order to induce eddy current in the human or animal body, as discussed in detail above. The transmitter 76 may, for example, be implemented using any one of the coils 22, 32 or 42 described above.

Figure 8 shows a data signal, indicated generally by the reference numeral 100, transmitted in operation 94 in an example embodiment. The data signal is a 1MHz carrier signal that is modulated with low frequency signals in order to transfer data using the human or animal body (e.g. the body 24 or 34) as a volume conductor. In the specific example signal 100, the 1MHz signal is modulated using a 2.5 kHz data signal having either a low amplitude (to transmit a logic '0') or a high amplitude (to transmit a logic '1'). Thus, the signal 100 transmits the data signal 0101010101.

It should be noted that the use of a 1 MHz signal is described by way of example only; other frequencies may be used. In some example embodiments, the signal frequency may be selected to achieve sufficient eddy current generation and avoid excessive attenuation within the body.

The signal 100 is, of course, one of many example data transmission formats that could be used. For example, data could be transmitted using amplitude modulation, frequency modulation, phase modulation or any other modulation of a carrier signal. Other transmission schemes are possible. Moreover, the transmission path could incorporate coded data transmission for added security. Spread-spectrum or other techniques may be used for improved signal-to-noise performance and for security purposes.

Some other transmission arrangements that could be used include using different carrier frequencies to transmit different signals. A system in accordance with the principles described herein could include additional channels. For example one or more high frequency wireless (e.g. Bluetooth) channels could be provided for the transfer of some data (e.g. data requiring higher data rates), with the in-body transmission described herein used for other data transmission (e.g. low data speed information). An example of low data speed information might be a security code used to encode the higher data rate signal.

At operation 96 of the algorithm 90, the receiver 78 is used to detect the potentials generated in response to the current induced in the human or animal body. As described above, the receiver 78 may be implemented using three galvanic or capacitive electrodes in a triangle shape so that voltage in any direction can be measured. Many other receiver pick-up arrangements could be used. A technical effect of the use of capacitive electrodes is that problems with skin irritation (e.g. allergic reactions) that may be present with contacting electrodes can be avoided. This may be of particularly relevant in long-term applications of the principles described herein (e.g. for long-term health monitoring). Another technical effect of example embodiments is that connection problems caused by walking, sleeping or other human or animal movements and activities may also be at least partially prevented by the use of capacitive coupling arrangements in the receiver.

At operation 98, the potentials detected by the receiver 78 are amplified (by amplifier 80) and used in some way (for example by being passed to the output 82). For example, the data sent from one or more sensors could be recorded and/or displayed to a user (such as an operator).

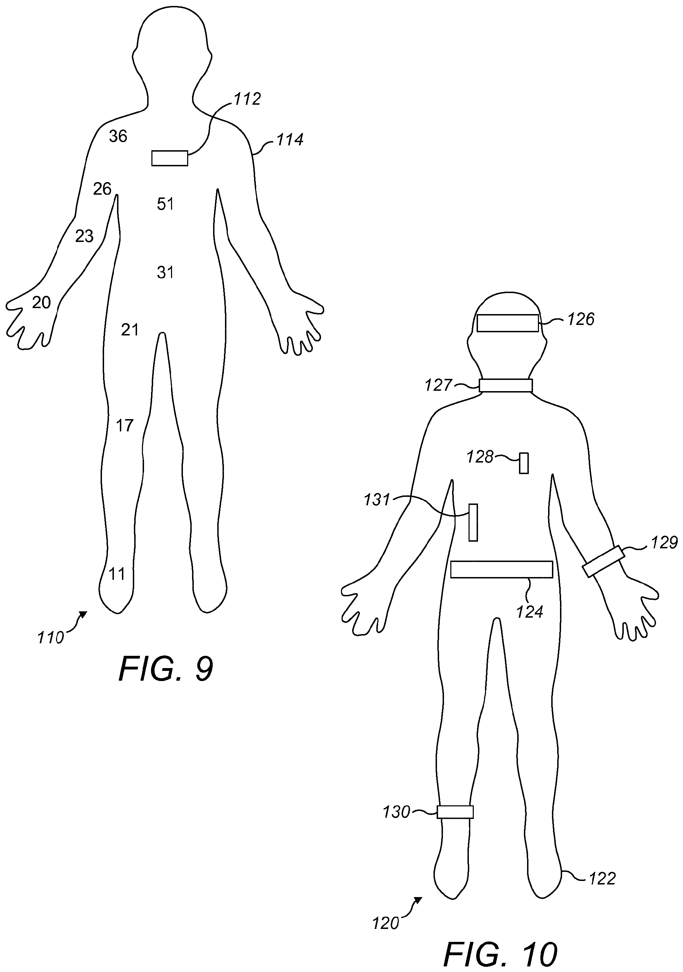

Figure 9 is a diagram, indicated generally by the reference numeral 110 showing an example embodiment in which a transmitter 112 was used to transmit data using a human body 114 as a volume conductor. A receiver was used to detect the amplitude of the received signals at various points around the body 114. The measurement results are indicated in the Figure. The measured signals are consistent with the modelling described above with reference to Figures 4 and 5.

Figure 10 is a diagram, indicated generally by the reference numeral 120, demonstrating example applications of the principles described herein.

The diagram 120 shows a human body 122. A master unit 124 is provided around the waist of the body 122. The master unit 124 includes a receiver for receiving data from a number of data sources provided on the body 122.

The data sources could include any combination of the following:
- One or more electroencephalography (EEG) sensors 126 to measure electrical signals within the brain;
- One or more balance sensor(s) 127;
- One or more electrocardiography (ECG), respiration or galvanic skin response (GSR) sensor(s) 128;
- One or more pulse monitor(s) 129;
- One or more movement or stride length sensor(s) 130; and
- One or more implanted sensor(s) 131.

The data sources 126 to 131 described above with reference to the diagram 120 are described by way of example. Some or all of the data sources may be omitted and other data sources may be provided in other example embodiments. For example, one or more action sensors, such as push-buttons or other switches may be provided.

Some or all of the data sources (such as data sources 126 to 131) may be used to direct a variable magnetic field into the body 122 for inducing a current for detection at the master unit 124.

One or more of the data sources 126 to 131 (and/or other data sensors) may be integrated within clothing. This may be particularly convenient when long-term monitoring of data is desired (e.g. for long-term patient monitoring or for wellness trackers, such as pulse rate monitors and movement sensors).

Since data is transferred through the body, it is not essential for the sensors and transmitters to be outside the body. Hence, one or more implanted sensors (such as the sensor 131) may be provided. One example of a useful implanted sensor is a blood glucose monitor. Other suitable sensors may be provided. Alternatively, or in addition, one or more receivers may be implanted. For example, a system for the delivery of painkiller medication or neural stimulation may include an implant receiver. Furthermore, an implanted transmitter or receiver may be only partially implanted. A transmitter on the body may, for example, transmit action information (e.g. a push button may be provided for the activation of the dosing of medication or stimulation pulses).

In the example embodiments described above, coils have been used for providing a variable magnetic field directed towards a human or animal body. This is not essential. Other arrangements for generating a variable magnet field could be used. These might include, for example, movable magnets (e.g. permanent magnets) or the use of a ferrite rod that axially directs a magnetic field into the body.

The variable magnetic field directed towards the human or animal body may be an oscillating magnetic field, but this is not essential. For example, the variable magnetic field may take the form of pulses or transients, or some other variation.

Furthermore, some of the example embodiments have been described above with reference to a human body. This is not essential. For example, an animal body could be used as a volume conductor.

The example embodiments described herein are generally related to health and wellness monitoring. This is not essential. For example, a transmitter could be used to generate a magnetic field that encodes identification information for a person. That information could be transmitted through the body using induced eddy currents. The person could use the data encoded within the induced current for identification purposes. By way of example, a person could apply their hands to electrode plates and a receiver at the electrode plates could identify the user (for example to allow entry into a secure environment).

Example embodiments described herein direct magnetic fields towards a human or animal body. In some example embodiments, care may be required to reduce the possibility of eavesdropping on transmitted signals by other parties. The design of transmitters can be such that the presence of stray magnetic fields is minimised (e.g. by controlling the strength of generated magnetic fields in different directions). The magnetic fields may also be provided at relatively low power (e.g. at a power as low as is feasible in a particularly implementation). Moreover, the data input may be encoded prior to transmission to make eavesdropping more difficult. In one example embodiment, for example, data is transmitted wirelessly outside the body in an encrypted form, with an encryption key being transmitted within the body.

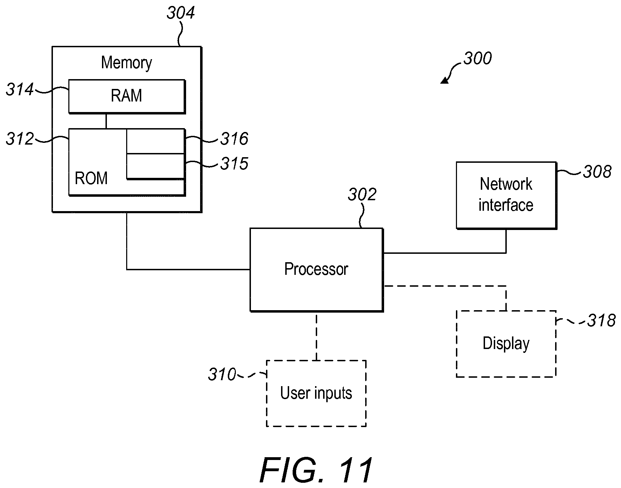

For completeness, Figure 11 is a schematic diagram of components of one or more of the example embodiments described previously (e.g. implementing some or all of the operations of the algorithm 90 described above, such as receiving or generating a signal for transmission or detecting and amplifying a received signal), which hereafter are referred to generically as processing systems 300. A processing system 300 may have a processor 302, a memory 304 closely coupled to the processor and comprised of a RAM 314 and ROM 312, and, optionally, user input 310 and a display 318. The processing system 300 may comprise one or more network interfaces 308 for connection to a network, e.g. a modem which may be wired or wireless.

The processor 302 is connected to each of the other components in order to control operation thereof.

The memory 304 may comprise a non-volatile memory, such as a hard disk drive (HDD) or a solid state drive (SSD). The ROM 312 of the memory 314 stores, amongst other things, an operating system 315 and may store software applications 316. The RAM 314 of the memory 304 is used by the processor 302 for the temporary storage of data. The operating system 315 may contain code which, when executed by the processor implements aspects of the algorithm 90 described above.

The processor 302 may take any suitable form. For instance, it may be a microcontroller, a plurality of microcontrollers, a processor, or a plurality of processors.

The processing system 300 may be a standalone computer, a server, a console, or a network thereof.

In some example embodiments, the processing system 300 may also be associated with external software applications. These may be applications stored on a remote server device and may run partly or exclusively on the remote server device. These applications may be termed cloud-hosted applications. The processing system 300 may be in communication with the remote server device in order to utilize the software application stored there.

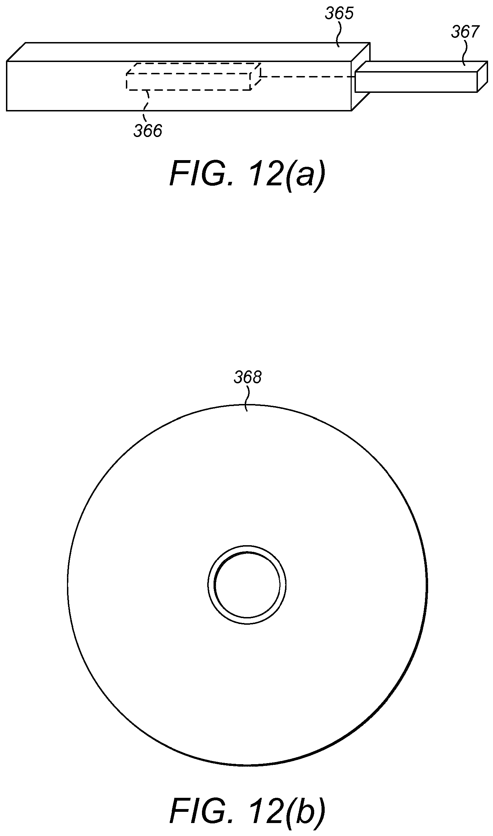

Figures 12a and 12b show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The memory 366 may be accessed by a computer system via a connector 367. The CD 368 may be a CD-ROM or a DVD or similar. Other forms of tangible storage media may be used.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices and other devices. References to computer program, instructions, code etc. should be understood to express software for a programmable processor firmware such as the programmable content of a hardware device as instructions for a processor or configured or configuration settings for a fixed function device, gate array, programmable logic device, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein may be performed in a different order and/or concurrently with each other. Furthermore, if desired, one or more of the above-described functions may be optional or may be combined. Similarly, it will also be appreciated that the flow diagrams of Figure 7 are examples only and that various operations depicted therein may be omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

It is also noted herein that while the above describes various examples, these descriptions should not be viewed in a limiting sense. Rather, there are several variations and modifications which may be made without departing from the scope of the present invention as defined in the appended claims.

## Claims

1. A method comprising:
generating a signal for transmission, wherein the signal for transmission is based on an input signal received from one or more sensors, wherein generating the signal for transmission comprises encoding the input signal;
converting the signal for transmission into a variable magnetic field directed towards a human or animal body, wherein the variable magnetic field induces eddy currents in order to generate an electrical current signal in the human or animal body, wherein the electrical current signal comprises data that is transmitted through the human or animal body; and
detecting the induced eddy currents with a suitable receiver circuit.

2. A method as claimed in claim 1, wherein the variable magnetic field is generated using a magnetic coil.

3. A method as claimed in claim 1 or claim 2, wherein the variable magnetic field is an oscillating magnetic field.

4. A method as claimed in any preceding claim, further comprising:
using receiver electrodes worn on the human or animal body to measure an electrical potential difference in the human or animal body, the electrical potential difference being indicative of the electrical current signal.

5. A method as claimed in claim 4, further comprising amplifying the measured electrical potential difference.

6. An apparatus comprising:
an input for receiving an input signal from one or more sensors; and
a transmitter circuitry for encoding the input signal;
a transmitter for converting the encoded input signal into a variable magnetic field, wherein, in use, the variable magnetic field induces eddy currents in order to generate an electrical current signal in a human or animal body, wherein the electrical current signal comprises data that is transmitted through the human or animal body; and
a receiver circuitry for detecting the induced eddy currents.

7. An apparatus as claimed in claim 6, further comprising the one or more sensors for providing the input signal.

8. An apparatus as claimed in claim 7, wherein the one or more sensors comprises one or more of a heart rate sensor, a blood pressure sensor and an oxygen level sensor.

9. An apparatus as claimed in any one of claims 6 to 8, wherein the apparatus is configured to be worn on the human or animal body.

10. An apparatus as claimed in any one of claims 6 to 9, wherein the apparatus is configured to be either totally or partially implanted inside the human or animal body.

11. An apparatus as claimed in any one of claims 6 to 10, further comprising a receiver comprising receiver electrodes configured to measure an electrical potential difference in the human or animal body, wherein the electrical potential difference is indicative of the electrical current signal.

12. An apparatus as claimed in claim 11, wherein the receiver electrodes are capacitive electrodes.

## Patentansprüche

1. Verfahren, das Folgendes umfasst:
Erzeugen eines Signals zur Übertragung, wobei das Signal zur Übertragung auf einem Eingangssignal basiert, das von einem oder mehreren Sensoren empfangen wird, wobei das Erzeugen des Signals zur Übertragung ein Codieren des Eingangssignals umfasst;
Umwandeln des Signals zur Übertragung in ein variables Magnetfeld, das auf einen Menschen- oder einen Tierkörper gerichtet wird, wobei das variable Magnetfeld Wirbelströme induziert, um ein elektrisches Stromsignal im Menschen- oder im Tierkörper zu erzeugen, wobei das elektrische Stromsignal Daten umfasst, die über den Menschen- oder den Tierkörper übertragen werden; und
Detektieren der induzierten Wirbelströme mit einer geeigneten Empfängerschaltung.

2. Verfahren nach Anspruch 1, wobei das variable Magnetfeld unter Verwendung einer Magnetspule erzeugt wird.

3. Verfahren nach Anspruch 1 oder Anspruch 2, wobei das variable Magnetfeld ein oszillierendes Magnetfeld ist.

4. Verfahren nach einem der vorhergehenden Ansprüche, das ferner Folgendes umfasst:
Verwenden von Empfängerelektroden, die am Menschen- oder am Tierkörper getragen werden, um eine elektrische Potenzialdifferenz im Menschen- oder im Tierkörper zu messen, wobei die elektrische Potenzialdifferenz das elektrische Stromsignal anzeigt.

5. Verfahren nach Anspruch 4, das ferner das Verstärken der gemessenen elektrischen Potenzialdifferenz umfasst.

6. Einrichtung, die Folgendes umfasst:
einen Eingang zum Empfangen eines Eingangssignals von einem oder mehreren Sensoren; und
eine Senderschaltung zum Codieren des Eingangssignals;
einen Sender zum Umwandeln des codierten Eingangssignals in ein variables Magnetfeld, wobei das variable Magnetfeld im Gebrauch Wirbelströme induziert, um ein elektrisches Stromsignal in einem Menschen- oder einem Tierkörper zu erzeugen, wobei das elektrische Stromsignal Daten umfasst, die über den Menschen- oder den Tierkörper übertragen werden; und
eine Empfängerschaltung zum Detektieren der induzierten Wirbelströme.

7. Einrichtung nach Anspruch 6, die ferner den einen oder die mehreren Sensoren zum Bereitstellen des Eingangssignals umfasst.

8. Einrichtung nach Anspruch 7, wobei der eine oder die mehreren Sensoren eines oder mehreres von einem Herzfrequenzsensor, einem Blutdrucksensor und einem Sauerstoffpegelsensor umfassen.

9. Einrichtung nach einem der Ansprüche 6 bis 8, wobei die Einrichtung dazu ausgelegt ist, am Menschen- oder am Tierkörper getragen zu werden.

10. Einrichtung nach einem der Ansprüche 6 bis 9, wobei die Einrichtung dazu ausgelegt ist, entweder ganz oder teilweise in den Menschen- oder den Tierkörper implantiert zu sein.

11. Einrichtung nach einem der Ansprüche 6 bis 10, die ferner einen Empfänger umfasst, der Empfängerelektroden umfasst, die dazu ausgelegt sind, eine elektrische Potenzialdifferenz im Menschen- oder im Tierkörper zu messen, wobei die elektrische Potenzialdifferenz das elektrische Stromsignal anzeigt.

12. Einrichtung nach Anspruch 11, wobei die Empfängerelektroden kapazitive Elektroden sind.

## Revendications

1. Procédé comprenant :
la génération d'un signal pour la transmission, dans lequel le signal pour la transmission est basé sur un signal d'entrée reçu d'un ou plusieurs capteurs, dans lequel la génération du signal pour la transmission comprend le codage du signal d'entrée ;
la conversion du signal pour la transmission en un champ magnétique variable dirigé vers un corps humain ou animal, dans lequel le champ magnétique variable induit des courants de Foucault afin de générer un signal de courant électrique dans le corps humain ou animal, dans lequel le signal de courant électrique comprend des données transmises à travers le corps humain ou animal ; et
la détection des courants de Foucault induits avec un circuit récepteur approprié.

2. Procédé selon la revendication l, dans lequel le champ magnétique variable est généré à l'aide d'une bobine magnétique.

3. Procédé selon la revendication 1 ou la revendication 2, dans lequel le champ magnétique variable est un champ magnétique oscillant.

4. Procédé selon l'une des revendications précédentes, comprenant en outre :
l'utilisation d'électrodes réceptrices portées sur le corps humain ou animal pour mesurer une différence de potentiel électrique dans le corps humain ou animal, la différence de potentiel électrique étant indicative du signal de courant électrique.

5. Procédé selon la revendication 4, comprenant en outre l'amplification de la différence de potentiel électrique mesurée.

6. Appareil comprenant :
une entrée pour recevoir un signal d'entrée d'un ou plusieurs capteurs ; et
un ensemble de circuits émetteur pour coder le signal d'entrée ;
un émetteur pour convertir le signal d'entrée codé en un champ magnétique variable, dans lequel, en cours d'utilisation, le champ magnétique variable induit des courants de Foucault afin de générer un signal de courant électrique dans un corps humain ou animal, dans lequel le signal de courant électrique comprend des données transmises à travers le corps humain ou animal ; et
un ensemble de circuits récepteur pour détecter les courants de Foucault induits.

7. Appareil selon la revendication 6, comprenant en outre les un ou plusieurs capteurs pour fournir le signal d'entrée.

8. Appareil selon la revendication 7, dans lequel les un ou plusieurs capteurs comprennent un ou plusieurs parmi un capteur de fréquence cardiaque, un capteur de pression artérielle et un capteur de niveau d'oxygène.

9. Appareil selon l'une des revendications 6 à 8, dans lequel l'appareil est configuré pour être porté sur le corps humain ou animal.

10. Appareil selon l'une des revendications 6 à 9, dans lequel l'appareil est configuré pour être totalement ou partiellement implanté dans le corps humain ou animal.

11. Appareil selon l'une des revendications 6 à 10, comprenant en outre un récepteur comprenant des électrodes réceptrices configurées pour mesurer une différence de potentiel électrique dans le corps humain ou animal, dans lequel la différence de potentiel électrique est indicative du signal de courant électrique.

12. Appareil selon la revendication 11, dans lequel les électrodes réceptrices sont des électrodes capacitives.
